# EUROPÄISCHE PATENTSCHRIFT

(11) **EP 1 446 180 B1**
(45) Veröffentlichungstag und Bekanntmachung des Hinweises auf die Patenterteilung: **05.07.2006**
(21) Anmeldenummer: 01274869.5
(22) Anmeldetag: 22.11.2001
(51) Int. Cl.: A61M 15/00, A61M 15/08

(54) **ATEMZUGSKONTROLLIERTE INHALATIONSVORRICHTUNG FÜR TROCKENPULVER**
INHALATION DEVICE FOR DRY POWDER CONTROLLED BY INSPIRATION
DISPOSITIF D'INHALATION DE POUDRE SECHE CONTROLE PAR INSPIRATION

(43) Veröffentlichungstag der Anmeldung: 18.08.2004
(73) Patentinhaber: Goldemann, Raul, 71700 Modlin (IL); Eckardt, Angela, 01309 Dresden (DE); Frydling, Ofer, 14169 Berlin (DE)
(72) Erfinder: GOLDEMANN, Raul, 71700 Modlin (IL)
(74) Vertreter: Hudler, Frank
(86) Internationale Anmeldenummer: PCT/DE2001/004377
(87) Internationale Veröffentlichungsnummer: WO 2003/047670

(56) Entgegenhaltungen:
- WO-A-96/22802
- WO-A-98/34662
- WO-A-98/53869
- US-A- 4 265 236
- US-A- 5 239 991
- US-A- 5 562 918

## Beschreibung

Die Erfindung betrifft eine atemzügkvntrollierte Inhalationsvorrichtung für Trockenpulver mit einer aus einem im wesentlichen zylindrischen Grundkörper bestehenden Luftführungseinheit, wobei die Luftführungseinheit einen abwechselnd mit Verengungen und jeweils nachfolgenden Erweiterungen versehenen Strömungskanal aufweist, wobei die verengungen und die Erweiterungen kontinuierlich ineinander übergehen, so dass der Strömungskanal eine dreidimensionale mäanderähnliche Form besitzt.

Die Inhalation als sanfte Heilmethode ist heute vor allem im Bereich der Atemwegserkrankungen, oder zur einfachen und schnell wirkenden Verabreichung von Medikamenten von großer Bedeutung. Dabei kann beispielsweise mit Medikamenten versetztes Trockenpulver als feiner Nebel den Atemwegen des Patienten zugeführt werden. Neben der Voraussetzung, dass während der Inhalation eine vollständige und gleichmäßige Verteilung des Trockenpulvers gewährleistet wird, ist vor allem auch eine rasche und für den Patienten unkomplizierte Nutzung der Inhalations- ' vorrichtung von Bedeutung. Aus der deutschen Patentanmeldung 199 48 289.6 ist ein Gerät bekannt, das durch den stromungsbeeinflussenden Aufbau eines einfachen zylindrischen Grundkörpers in einem speziellen Inhalationsgehäuse eine entsprechende Verteilung des Trockenpulvers ermöglicht. Allerdings ist die bei dieser Erfindung verwendete Luftführungseinheit in Gestalt eines zylindrischen Grundkörpers von einem Gehäuse umgeben und nicht flexibel austauschbar. Weiterhin muss das Trockenpulver einer speziellen vorratskammer zugeführt werden und gelangt erst von dort in die Luftführungseinheit. Um eine exakte Dosierung des Trockenpulvers zu garantieren und um sicherzustellen, dass das Trockenpulver keine Feuchtigkeit annimmt, ist ein erheblicher technischer Aufwand erforderlich.

Aus der US-A-5 562 918 geht ein Dispensersystem hervor, mit dem pulverförmige Medikamente durch Einsaugen mittels Atemluft inhaliert werden können. Dieser Dispenser besteht aus mehreren Einzelteilen, insbesondere einem zylindrischen Hauptkörper, der mittels axialer Krafteinwirkung auftrennbare Verschlüsse ausweist und mit einer Ansaugöffnung und einem Lufteinlass versehen ist. Um eine ausreichende Verwirbelung der durch den Dispenser gesaugten Luft zu erreichen, ist im Lufteinlass eine Turbine vorgesehen. Im Mundstück ist ferner ein Grill vorgesehen, der das Ansaugen von größeren Partikeln verhindern soll.

Die WO-A-96/22802 zeigt einen Inhalator, der einen röhren förmigen Körper zur Definition eines Luftdurchlasses aufweist, mit Verschlusskappen versehen und mit einem Pulver befüllt ist. Der Luftdurchlass kann im Querschnitt rechteckig, quadratisch, polygonal, elliptisch oder kreisrund sein, wobei die Querschnittssfläche am Mundstück geringer ist, als am gegenüber liegenden Ende. Zusätzlich sind biegbare Abschnitte vorgesehen, um eine Anpassung an antomische Gegebenheiten zu ermöglichen. Um die verteilung der Partikel beim Inhalieren zu verbessern, kann der Inhalator mit Mitteln zur Erzeugung einer Rotation der Luft versehen sein. Das können spiralförmig verlaufende vertiefungen oder Rippen an der Innenwand des röhrenförmigen Körpers sein.

Da die ständige Funktionsbereitschaft solcher medizinischer Geräte von großer Bedeutung ist, muss stetig versucht werden, durch eine Vereinfachung des technischen Aufbaus, Störungsrisiken zu minimieren.

Der Erfindung liegt demnach die Aufgabe zugrunde, eine Inhalationsvorrichtung zu schaffen, die einen sekundenschnellen Zugriff durch sofortige Einsatzbereitschaft ermöglicht und durch eine aufbaubedingte Reduzierung der Vorrichtungselemente die Bedienschritte des Patienten vor, während und nach der Inhalation auf ein Minimum beschränkt, wobei ebenfalls aufbaubedingt ein Störungsrisiko der Vorrichtung nahezu ausgeschlossen wird.

Diese Aufgabe wird, ausgehend von einer atemzugkontrollierten Inhalationsvorrichtung der eingangs genannten Art, erfindungsgemäß dadurch gelöst, dass mindestens zwei magazinartig miteinander verbundene zylindrischen Grundkörper (1) als Luftführungseinheiten vorgesehen sind, wobei die Grundkörper (1) über Sollbruchstellen (8 und 9) mittelbar oder unmittelbar miteinander verbunden sind, dass an jedem-Grundkörper (1) ein Lufteinlass (2) und eine Ansaugöffnung (3) vorgesehen sind, die gleichsinnig ausgerichtet und im Transportzustand jeweils mit einem Originalitätsverschluss (5a, 5b) abgedichtet sind, und dass die Grundkörper (1) mit einer rationierten Menge Trockenpulver gefüllt sind.

Dem Patienten steht mit einer erfindungsgemäßen Vorrichtung der beschriebenen Art eine extrem kostengünstige und sofort einsatzfähige Möglichkeit zur Verfügung, rationierte wirkstoffe im Bedarfsfall zu inhalieren, ohne dabei einem Störungsrisiko der Inhalationsvorrichtung ausgesetzt zu sein, da sich der Wirkstoff bereits in den magazinierten und leicht von einander zu lösenden Luftführungseinheiten befindet und ansonsten die Inhalationsvorrichtung selbst in oben beschriebener Weise technisch minimiert wurde. Darüber hinaus kommt die erfindungsgemäße Vorrichtung vollkommen ohne bewegliche Einzelteile aus und kann somit sehr kostengünstig hergestellt und als Wegwerfartikel eingesetzt werden. Außerdem wird hierdurch das Eindringen von Feuchtigkeit sicher verhindert.

Durch die Versiegelung der Inhalationsvorrichtung über Originalitätsverschlüsse wird gewährleistet, dass stets nur die vorbestimmte Menge des bereits in der Luftführungseinheit befindlichen wirkstoffes bzw. Trockenpulvers zur Verfügung steht.

Diese Originalitätsverschlüsse sind nach einer Ausführungsform der Erfindung unproblematisch über vorgestanzte Sollbruchstellen abtrennbar.

Darüber hinaus sieht eine Ausführungsform der Erfindung vor, dass der jeweilige Originalitätsverschluss mit einem flachen geometrischen Griffteil fest verbunden ist. Das Griffteil dient hierbei der Handlichkeit beim Abtrennen des originalitätsverschlusses.

Nach weiteren Ausführungen sind der Lufteinlass und die Ansaugöffnung in einer ihrem Umriss entsprechenden Aussparung des jeweiligen Griffteils liegend, mit dem jeweiligen Griffteil über Sollbruchstellen verbunden, wobei die Griffteile in der Symmetrieebene der Magazinierung angeordnet sind und Sollbruchstellen zwischen den jeweiligen Griffteilen und dem zylindrischen Grundkörpern vorgesehen sind.

In diesen Ausführungsformen kommt den Griffteilen zusätzlich die Funktion eines geometrisch-symmetrischen Bindegliedes zwischen den einzelnen über Sollbruchstellen mittelbar oder unmittelbar verbundenen zylindrischen Grundkörpern zu. Außerdem stabilisieren sie die Integrität des Originalitätsverschlusses.

Durch die in der angegebenen Weise vorgesehenen Sollbruchstellen können die zylindrischen Grundkörper zunächst voneinander getrennt und anschließend die Ansaugöffnung und der Lufteinlass von den Griffteilen vollständig befreit werden.

Bei einer besonderen Ausführungsform der Erfindung sind die zylindrischen Grundkörper innerhalb der Magazinierung als Paare angeordnet, wobei die zylindrischen Grundkörper innerhalb eins Paares über Sollbruchstellen an den Außenkanten der Griffteile des Lufteinlasses miteinander verbunden sind.

Weiterhin sind diese Paare wiederum durch Sollbruchstellen jeweils an den den Griffteilen des Lufteinlasses gegenüberliegenden Außenkanten der zylindrischen Grundkörper miteinander verbunden. Die hier gewählten Anordnung der zylindrischen Grundkörper innerhalb der Magazinierung stellt eine besonders günstige Verbindungsvariante dar, die sich durch eine hohe Stabilität auszeichnet und innerhalb des Herstellungsprozesses dem Auffüllen der zylindrischen Grundkörper mit dem Trockenpulver und den sich anschließenden Verbindungs- und Abdichtungsschritten dienlich ist. Andere Anordnungsvarianten sind denkbar.

In einer besonders vorteilhaften Ausführungsform der Erfindung ist die Sollbruchstelle zum Abtrennen des Originalitätsverschlusses am Lufteinlass fester, als die Sollbruchstellen zwischen Lufteinlass und Griffteil und die Sollbruchstelle zwischen Griffteil und zylindrischem Grundkörper. Außerdem ist die Sollbruchstelle zum Abtrennen des Originalitätsverschlusses an der Ansaugöffnung fester, als die Sollbruchstelle zwischen Ansaugöffnung und Griffteil und die Sollbruchstelle zwischen Griffteil und zylindrischem Grundkörper.

Dadurch wird gewährleistet, dass der Originalitätsverschluss nicht versehentlich geöffnet wird.

Schließlich ist in einer weiteren günstigen Ausführungsform vorgesehen, dass die Sollbruchstelle zwischen Griffteil und zylindrischem Grundkörper fester ist als die Sollbruchstelle an der Außenkante des Griffteils und die Sollbruchstelle an der dem Griffteil des Lufteinlasses gegenüberliegenden Außenkante des zylindrischen Grundkörpers. Hierdurch wird vermieden, dass es nicht bereits mit dem Lösen einer einzelnen Luftführungseinheit aus der Magazinierung zu einem teilweisen oder vollständigen Lösen des Griffteils vom zylindrischen Grundkörper kommt und auch der Originalitätsverschluss in seiner durch das Griffteil erzeugten Stabilisierung gefährdet wird.

Die Erfindung soll nachfolgend an einem Ausführungsbeispiel näher erläutert werden.

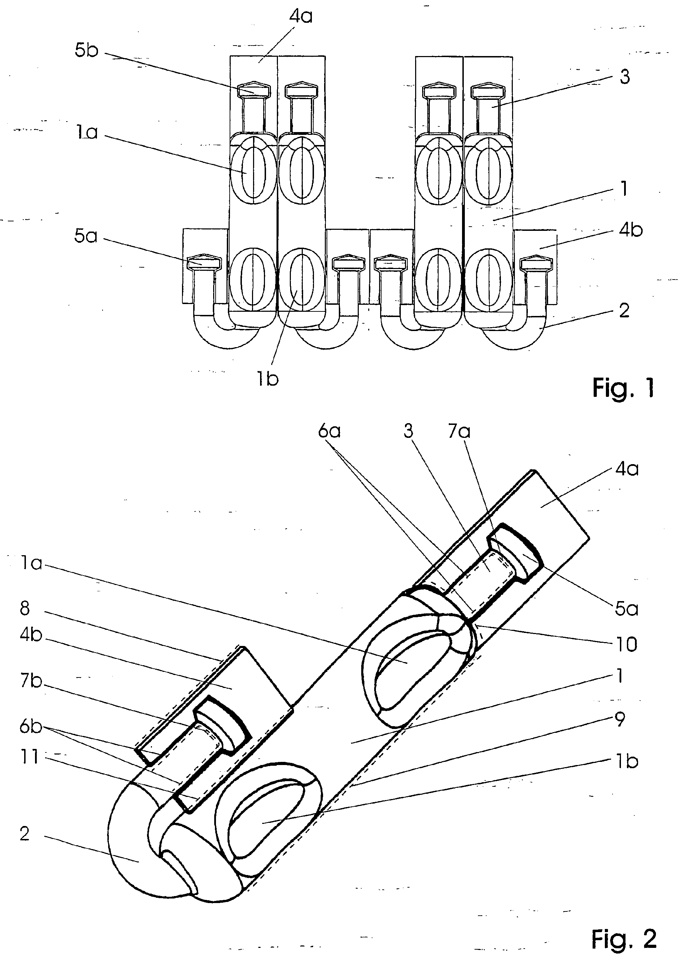

Hierbei zeigt die zugehörige Zeichnung in
- Fig. 1: die Inhalationsvorrichtung in einer möglichen Magazinierungsanordnung und
- Fig. 2: eine einzelne Luftführungseinheit in Form eines zylindrischen Grundkörpers.

Die erfindungsgemäße Inhalationsvorrichtung besteht aus einer Mehrzahl von zu einer Kette verbundenen zylindrischen Grundkörpern 1. Diese zylindrischen Grundkörper 1 sind als Luftführungseinheiten ausgestaltet, wobei sie einen Strömungskanal aufweisen, der abwechselnd mit Verengungen und jeweils nachfolgenden Erweiterungen versehen ist, wobei die Verengungen und die Erweiterungen kontinuierlich ineinander übergehen und der Strömungskanal für die durch die Luftführungseinheit strömende Luft in dreidimensionaler mäanderähnlicher Form ausgestaltet ist. Der zylindrische Grundkörper 1 ist dazu mit kugelkalottenähnlichen Einbuchtungen 1a und 1b versehen, die sich von jeweils gegenüberliegenden Wandungen des zylindrischen Grundkörpers 1 in den Strömungskanal erstrecken.

Der Patient kann im Bedarfsfall der Magazinierung eine einzelne Luftführungseinheit mit einer vorportionierten Menge an mit Medikamenten versetztem Trockenpulver oder einem Medikament in Form von Trockenpulver entnehmen. Die einzelnen Luftführungseinheiten in Gestalt der zylindrischen Grundkörper 1 sind unmittelbar oder über flache Griffteile 4b mittelbar durch vorgestanzte Sollbruchstellen 8 und 9 miteinander verbunden, so dass die benötigte Luftführungseinheit aus der Magazinierung herausgebrochen werden kann.

Als günstig in Bezug auf die für den Transport wichtige Stabilität der Vorrichtung hat sich dabei eine Anordnung dergestalt erwiesen, dass die zylindrischen Grundkörper innerhalb der Magazinierung als Paare angeordnet sind, wobei die zylindrischen Grundkörper 1 innerhalb eines Paares an den Außenkanten der Griffteile 4b der Lufteinlässe 2 miteinander verbunden sind. Diese Paare sind ihrerseits wiederum durch Sollbruchstellen 9 jeweils an den den Griffteilen 4b des Lufteinlasses 2 gegenüberliegenden Außenkanten der zylindrischen Grundkörper 1 miteinander verbunden.

Da die Sollbruchstelle 7b zum Abtrennen des Originalitätsverschlusses 5b am Lufteinlass 2 fester ist, als die Sollbruchstellen 6b zwischen Lufteinlass 2 und Griffteil 4b und die Sollbruchstelle 11 zwischen Griffteil 4b und zylindrischem Grundkörper 1 und außerdem die Sollbruchstelle 7a zum Abtrennen des Originalitätsverschlusses 5a an der Ansaugöffnung 3 fester ist, als die Sollbruchstelle 6a zwischen Ansaugöffnung 3 und Griffteil 4a und die Sollbruchstelle 10 zwischen Griffteil 4a und zylindrischem Grundkörper 1, wird gewährleistet, dass der Originalitätsverschluss 5a nicht versehentlich geöffnet wird.

Ein versehentliches Lösen des Griffteils 4b und damit eine Gefährdung der Stabilisierung des Originalitätsverschlusses 5b wird darüber hinaus dadurch vermieden, dass die Sollbruchstelle 11 zwischen Griffteil 4b und zylindrischem Grundkörper 1 fester ist als die Sollbruchstelle an der Außenkante des Griffteils 8 und die Sollbruchstelle 9 an der dem Griffteil 4b des Lufteinlasses 2 gegenüberliegenden Außenkante des zylindrischen Grundkörpers 1.

Um die von der Luftführungseinheit beinhaltete Menge Trockenpulver inhalieren zu können, werden die an einem Lufteinlass 2 und einer Ansaugöffnung 3 befindlichen und mit diesen ebenfalls über Sollbruchstellen 7a und 7b verbundenen Originalitätsverschlüsse 5a und 5b mittels der Griffteile 4a und 4b abgetrennt, wobei die Griffteile vollständig von den zylindrischen Grundkörpern durch Aufbrechen entsprechender Sollbruchstellen 6a, 6b, 10 und 11 beseitigt werden können und die Inhalationsvorrichtung somit schließlich in den gebrauchsbereiten Zustand versetzt werden kann. Daraufhin kann die Ansaugöffnung 3 der Inhalationsvorrichtung zum Mund geführt und Luft angesaugt bzw. inhaliert werden. Beim Ansaugen der über den Lufteinlass 2 einströmenden Luft kommt es dabei durch die beschriebene besondere Ausgestaltung des zylindrischen Grundkörpers 1 zu einer optimalen Verteilung der mit dem Wirkstoff versetzten Partikel des Trockenpulvers, indem die durchströmende Luft in spezieller Weise zum Zirkulieren angeregt wird. Dadurch, dass Ansaugöffnung 3 und Lufteinlass 2 gleichsinnig - nach oben - ausgerichtet sind, und der Patient beim Inhalieren seinen Kopf um kaum mehr als 90 Grad neigen wird, ist ein ungewolltes Entweichen des Trockenpulvers nicht möglich.

### Bezugzeichenliste

- 1: zylindrischer Grundkörper
- 1a: kugelkalottenähnliche Einbuchtungen
- 1b: kugelkalottenähnliche Einbuchtungen
- 2: Lufteinlass
- 3: Ansaugöffnung
- 4a: Griffteil
- 4b: Griffteil
- 5a: Originalitätsverschluss
- 5b: Originalitätsverschluss
- 6a: Sollbruchstelle
- 6b: Sollbruchstelle
- 7a: Sollbruchstelle
- 7b: Sollbruchstelle
- 8: Sollbruchstellen
- 9: Sollbruchstelle
- 10: Sollbruchstelle
- 11: Sollbruchstelle

## Patentansprüche

1. Atemzugkontrollierte Inhalationsvorrichtung für Trockenpulver mit einer aus einem im wesentlichen zylindrischen Grundkörper bestehenden Luftführungseinheit, wobei die Luftführungseinheit einen abwechselnd mit Verengungen (1a, 1b) und jeweils nachfolgenden Erweiterungen versehenen Strömungskanal aufweist, wobei die Verengungen und die Erweiterungen kontinuierlich ineinander übergehen, so dass der Strömungskanal eine dreidimensionale mäanderähnliche Form besitzt, **dadurch gekennzeichnet, dass** mindestens zwei magazinartig miteinander verbundene zylindrischen Grundkörper (1) als Luftführungseinheiten vorgesehen sind, wobei die Grundkörper (1) über Sollbruchstellen (8 und 9) mittelbar oder unmittelbar miteinander verbunden sind, dass an jedem Grundkörper (1) ein Lufteinlass (2) und eine Ansaugöffnung (3) vorgesehen sind, die gleichsinnig ausgerichtet und im Transportzustand jeweils mit einem Originalitätsverschluss (5a, 5b) abgedichtet sind, und dass die Grundkörper (1) mit einer rationierten Menge Trockenpulver gefüllt sind.

2. Atemzugkontrollierte Inhalationsvorrichtung nach Anspruch 1 **dadurch gekennzeichnet, dass** die Origirialitätsverschlüsse (5a und 5b) mit dem Lufteinlass (2) und der Ansaugöffnung (3) über Sollbruchstellen (7a und 7b) verbunden sind.

3. Atemzugkontrollierte Inhalationsvorrichtung nach Anspruch 1 und 2 **dadurch gekennzeichnet, dass** die Originalitätsverschlüsse (5a und 5b) mit je einem flachen Griffteil (4a und 4b) fest verbunden sind.

4. Atemzugkontrollierte Inhalationsvorrichtung nach Anspruch 3 **dadurch gekennzeichnet, dass** die Griff teile (4a und 4b) in der symmetrieebene der Magazinierung angeordnet sind.

5. Atemzugkontrollierte Inhalationsvorrichtung nach Anspruch 3 **dadurch gekennzeichnet, dass** der Lufteinlass (2) in einer dem Umriss des Lufteinlasses (2) entsprechenden Aussparung des Griffteils (4b) liegend, mit dem Griffteil (4b) über Sollbruchstellen (6b) verbunden ist und darüber hinaus eine Verbindung in Form einer Sollbruchstelle (11) zwischen dem Griffteil und dem zylindrischen Grundkörper besteht.

6. Atemzugkontrollierte Inhalationsvorrichtung nach Anspruch 3 **dadurch gekennzeichnet, dass** die Ansaugöffnung (3) in einer dem Umriss der Ansaugöffnung (3) entsprechenden Aussparung des Griffteils (4a) liegend, mit dem Griffteil (4a) über Sollbruchstellen (6a) verbunden ist und darüber hinaus eine Verbindung in Form einer Sollbruchstelle (10) zwischen dem Griffteil (4a) und dem zylindrischen Grundkörper (1) besteht.

7. Atemzugkontrollierte Inhalationsvorrichtung nach Anspruch 3 **dadurch gekennzeichnet, dass** die zylindrischen Grundkörper (1) innerhalb der magazinartigen Anordnung als Paare angeordnet sind, wobei die zylindrischen Grundkörper (1) über Sollbruchstellen (8) an den Außenkanten der Griffteile (4b) der Lufteinlässe (2) miteinander verbunden sind.

8. Atemzugkontrollierte Inhalationsvorrichtung nach Anspruch 7 **dadurch gekennzeichnet, dass** die Paare durch Sollbruchstellen (9) jeweils an den den Griffteilen, (4b) des Lufteinlasses (2) gegenüberliegenden Außenkanten der zylindrischen Grundkörper (1) miteinander verbunden sind.

9. Atemzugkontrollierte Inhalationsvorrichtung nach Anspruch 2 **dadurch gekennzeichnet, dass** die Sollbruchstelle (7b) zum Abtrennen des Originalitätsverschlusses (5b) am Lufteinlass (2) fester ist, als die Sollbruchstellen (6b) zwischen Lufteinlass und Griffteil und eine Sollbruchstelle (11) zwischen Griffteil und zylindrischem Grundkörper.

10. Atemzugkontrollierte Inhalationsvorrichtung nach Anspruch 2 **dadurch gekennzeichnet, dass** die Sollbruchstelle (7a) zum Abtrennen des Originalitäts verschlusses (5a) an der Ansaugöffnung (3) fester ist, als die Sollbruchstelle (6a) zwischen Ansaugöffnung und Griffteil und die Sollbruchstelle (10) zwischen Griffteil und zylindrischem Grundkörper.

11. Atemzugkontrollierte Inhalationsvorrichtung nach Anspruch 9 und 10 **dadurch gekennzeichnet, dass** die Sollbruchstelle (11) zwischen Griff teil (4b) und zylindrischem Grundkörper (1) fester ist als die Sollbruchstelle an der Außenkante des Griff teils (8) und die Sollbruchstelle (9) an der dem Griffteil (4b) des Lufteinlasses (2) gegenüberliegenden Außenkante des zylindrischen Grundkörpers (1).

## Claims

1. Breathing-controlled inhalation device for dry powders, having an air guide unit consisting essentially of a cylindrical body, said air guide unit comprising a flow passage alternately provided with constrictions and in each instance following enlargements, said constrictions and enlargements passing continuously one into another, so that the flow passage is of three-dimensional meander-like conformation, **characterized in that** at least two cylindrical bodies (1) connected to each other magazine-fashion are provided as air guide units, the bodies (1) being directly or indirectly connected one to another by way of weak spots (8 and 9), **in that** each body (1) is provided with an air inlet (2) and an intake opening (3) oriented in like direction and, in transport condition, each sealed with an authenticity closure (5a, 5b), and **in that** the bodies (1) are filled with a rationed quantity of dry powder.

2. Breathing-controlled inhalation device according to claim 1, **characterized in that** the authenticity closures (5a and 5b) are connected to the air inlet (2) and the intake opening (3) by way of weak spots (7a and 7b).

3. Breathing-controlled inhalation device according to claims 1 and 2, **characterized in that** the authenticity closures (5a and 5b) are each fixedly connected to a flat geometrical grip part (4a and 4b).

4. Breathing-controlled inhalation device according to claims 1 to 3, **characterized in that** the grip parts (4a and 4b) are arranged in the plane of symmetry of the magazine.

5. Breathing-controlled inhalation device according to claim 3, **characterized in that** the air inlet (2), lying in a recess of the grip part (4b) matching the outline of the air inlet (2), is connected to the grip part (4b) by way of weak spots (6b), and furthermore there is a connection in the form of a weak spot (11) between the grip part and the cylindrical body.

6. Breathing-controlled inhalation device according to claim 3, **characterized in that** the intake opening (3), lying in a recess of the grip part (4a) matching the outline of the intake opening (3), is connected to the grip part (4a) by way of weak spots (6a), and furthermore there is a connection in the form of a weak spot (10) between the grip part (4a) and the cylindrical body (1).

7. Breathing-controlled inhalation device according to claim 3, **characterized in that** the cylindrical bodies (1) are arranged in pairs within the magazine-like arrangement, the cylindrical bodies (1) being connected to each other by way of weak spots (8) at the outer edges of the grip parts (4b) of the air inlets (2).

8. Breathing-controlled inhalation device according to claim 7, **characterized in that** the pairs are connected to each other in each instance by way of weak spots (9) at the outer edges of the cylindrical bodies (1) opposed to the grip parts (4b) of the air inlet (2).

9. Breathing-controlled inhalation device according to claims 2, **characterized in that** the weak spot (7b) for detaching the authenticity closure (5b) at the air inlet (2) is stronger than the weak spots (6b) between air inlet and grip part and the weak spot (11) between grip part and cylindrical body.

10. Breathing-controlled inhalation device according to claim 2, **characterized in that** the weak spot (7a) for detaching the authenticity closure (5a) at the intake opening (3) is stronger than the weak spot (6a) between intake opening and grip part and the weak spot (10) between grip part and cylindrical body.

11. Breathing-controlled inhalation device according to claims 9 to 10, **characterized in that** the weak spot (11) between grip part (4b) and cylindrical body (1) is stronger than the weak spot at the outer edge of grip part (8) and the weak spot (9) at the outer edge of the cylindrical body (1) opposed to the grip part (4b) of the air inlet (2).

## Revendications

1. Dispositif d'inhalation à inspiration contrôlée pour poudre sèche comportant une unité de guidage d'air constituée d'un corps de base sensiblement cylindrique, l'unité de guidage d'air présentant un canal d'écoulement pourvu en alternance de rétrécissements (1a, 1b) et d'élargissements venant ensuite, les rétrécissements et élargissements se raccordant les uns aux autres en continu, de sorte que le canal d'écoulement présente une forme tridimensionnelle en méandres, **caractérisé en ce qu'**au moins deux corps de base cylindriques (1) reliés l'un à l'autre à la manière d'un magasin sont prévus en tant qu'unité de guidage d'air, les corps de base (1) étant reliés entre eux directement ou indirectement par des emplacements de rupture imposée (8 et 9), **en ce que** sur chaque corps de base (1) sont prévues une entrée d'air (2) et une ouverture d'aspiration (3) qui sont orientées dans le même sens et qui sont étanches à l'état de transport chacune avec une fermeture d'origine (5a, 5b), et **en ce que** les corps de base (1) sont remplis avec une quantité rationnée de poudre sèche.

2. Dispositif d'inhalation à inspiration contrôlée selon la revendication 1, **caractérisé en ce que** les fermetures d'origine (5a et 5b) sont reliées à l'entrée d'air (2) et à l'ouverture d'aspiration (3) par emplacements de rupture imposée (7a et 7b).

3. Dispositif d'inhalation à inspiration contrôlée selon les revendications 1 et 2, **caractérisé en ce que** les fermetures d'origine (5a et 5b) sont solidaires chacune d'un élément de préhension (4a et 4b) plat.

4. Dispositif d'inhalation à inspiration contrôlée selon la revendication 3, **caractérisé en ce que** les éléments de préhension (4a et 4b) sont disposés dans le plan de symétrie des magasins.

5. Dispositif d'inhalation à inspiration contrôlée selon la revendication 3, **caractérisé en ce que** l'entrée d'air (2), située dans une découpe de l'élément de préhension (4b), correspondant au contour de l'entrée d'air (2), est reliée à l'élément de préhension (4b) par des emplacements de rupture imposée (6b), et il existe par ailleurs une liaison sous la forme d'un emplacement de rupture imposée (11) entre l'élément de préhension et le corps de base cylindrique.

6. Dispositif d'inhalation à inspiration contrôlée selon la revendication 3, **caractérisé en ce que** l'ouverture d'aspiration (3), située dans une découpe de l'élément de préhension (4a), correspondant au contour de l'ouverture d'aspiration (3), est reliée à l'élément de préhension (4a) par des emplacements de rupture imposée (6a), et il existe par ailleurs une liaison sous la forme d'un emplacement de rupture imposée (10) entre l'élément de préhension (4a) et le corps de base cylindrique (1).

7. Dispositif d'inhalation à inspiration contrôlée selon la revendication 3, **caractérisé en ce que** les corps de base cylindriques (1) sont disposés à l'intérieur du dispositif de type magasin sous la forme de paires, les corps de base cylindriques (1) étant reliés entre eux par des emplacements de rupture imposée (8) sur les bords extérieurs des éléments de préhension (4b) des entrées d'air (2).

8. Dispositif d'inhalation à inspiration contrôlée selon la revendication 7, **caractérisé en ce que** les paires sont reliées entre elles chacune par des emplacements de rupture imposée (9), sur les bords extérieurs, opposés aux éléments de préhension (4b) de l'entrée d'air (2), des corps de base cylindriques (1).

9. Dispositif d'inhalation à inspiration contrôlée selon la revendication 2, **caractérisé en ce que** l'emplacement de rupture imposée (7b), pour séparer la fermeture d'origine (5b) sur l'entrée d'air (2), est plus solide que les emplacements de rupture imposée (6b) entre l'entrée d'air et l'élément de préhension et l'emplacement de rupture imposée (11) entre l'élément de préhension et le corps de base cylindrique.

10. Dispositif d'inhalation à inspiration contrôlée selon la revendication 2, **caractérisé en ce que** l'emplacement de rupture imposée (7a) pour séparer la fermeture d'origine (5a) sur l'ouverture d'aspiration (3), est plus solide que l'emplacement de rupture imposée (6a) entre l'ouverture d'aspiration et l'élément de préhension et l'emplacement de rupture imposée (10) entre l'élément de préhension et le corps de base cylindrique.

11. Dispositif d'inhalation à inspiration contrôlée selon les revendications 9 et 10, **caractérisé en ce que** l'emplacement de rupture imposée (11) entre l'élément de préhension (4b) et le corps de base cylindrique (1) est plus solide que l'emplacement de rupture imposée sur le bord extérieur de l'élément de préhension (8) et l'emplacement de rupture imposée (9) sur le bord extérieur, opposé à l'élément de préhension (4b) de l'entrée d'air (2), du corps de base cylindrique.
